# EUROPEAN PATENT APPLICATION

(11) **EP 2 939 701 A1**
(43) Date of publication of application: **04.11.2015**
(21) Application number: 13867162.3
(22) Date of filing: 20.12.2013
(51) Int. Cl.: A61M 25/10

(54) **CATHETER BALLOON, CATHETER, AND METHOD OF MANUFACTURING CATHETER BALLOON**

(30) Priority: 25.12.2012 JP 2012281704
(71) Applicant: Tokai Medical Products, Inc., Aichi 486-0808 (JP)
(72) Inventor: TSUTSUI, Nobumasa, Kasugai-shi Aichi 486-0808 (JP); TSUTSUI, Yasuhiro, Kasugai-shi Aichi 486-0808 (JP); MURAKI, Yasuhiro, Kasugai-shi Aichi 486-0808 (JP)
(74) Representative: Zeuner Summerer Stütz
(86) International application number: PCT/JP2013/084196
(87) International publication number: WO 2014/103907

(57) **Abstract**

[Object] Provided is an expansive catheter balloon that has a spreadable outer diameter controllable easily in treatments for a calcific aortic stenosis, rheumatic and congenital aortic stenosis and others, and that can be effectively prevented from being freely shifted and can easily be fixed at a predetermined position.

[Solution] The catheter balloon 10 includes a cylindrical balloon part 20 that is made non-expansive or low-expansive, and a band part 30 made of a stretchable elastic body. When a stretching force is not applied, the band part 30 has a shorter diameter than a spread diameter of the balloon part 20 and is wound around the center of the balloon part 20.

## Description

### Technical Field:

The present invention relates to a catheter balloon, a catheter, and a method of manufacturing the catheter balloon.

### Background Art:

Causes for stenotic aortic lesion are, for example, an acquired calcific or rheumatic aortic stenosis, and a congenital aortic stenosis. For example, about the calcific aortic stenosis, at the time when the lesion thereof is deteriorated, the calcification of valve cusp advances so that the valve cusp gradually hardens. As a result, the mobile scope thereof is gradually restricted to cause cardiac insufficiency. For this reason, in the case of a severe valve cusp stenosis, it is necessary to treat the stenosis by operation.

In order to treat such a disease, balloon valvuloplasty is suggested as a low-invasive treating method for spreading a valve undergoing a stenosis, such as a calcific aortic stenosis, or a rheumatic or congenital aortic stenosis. An ordinary operation for the present treating method about the calcific aortic stenosis is as follows; first, a catheter having a deflated balloon is percutaneously inserted into a vein or artery, and then the balloon is arranged inside a cardiac valve required to be treated. Next, by spreading the balloon, the valve cusp is spread to pulverize a calcific portion depositing on the valve cusp. In this way, the softness of the valve cusp is restored to improve the mobile scope. After it is confirmed that the ejection quantity of the blood flow increases, the balloon is deflated and the catheter is removed from the inside of the body.

In order to spread the valve cusp of the aorta largely, the catheter balloon used in the present treatment may be made of a highly elastic material such as latex or silicone to cause the whole of the balloon to have expansibility. By using the highly elastic material to make the balloon expansible, advantages are produced that the initial outer diameter thereof can be made small and further the flexibility thereof facilitates the invasion and advance of the balloon into the vessel. Furthermore, the balloon has an advantage that when the balloon is removed from the blood vessel, the balloon can easily be withdrawn since the outer diameter of the balloon is made small after the deflation. However, the balloon made of the high elastic material such as latex or silicone can be arbitrarily made large in diameter to cause a problem of requiring an instrument or means for precisely controlling the spread diameter of the expansible balloon.

About any existing balloon, the shape thereof is cylindrical or ovoid, and further the balloon is easily slipped in blood or on a vascular wall. Thus, the balloon is freely shifted, and there remains a problem that a considerable skill and attentiveness are required to locate the balloon at a predetermined position.

### Citation List

### Patent Document

Patent document 1: JP 2005-537856 A

### Summary of the Invention

### Problems to be Solved

Thus, the present invention has been made in light of the above-mentioned problems, and an object thereof is to provide a catheter balloon, a catheter and a method of manufacturing the catheter balloon, the balloon being an expansible balloon that has a spreadable outer diameter controllable easily in treatments for a calcific aortic stenosis, rheumatic and congenital aortic stenosis and others, and that can be effectively prevented from being freely shifted and can easily be fixed at a predetermined position.

### Solution to Problem

In order to attain the object, the present invention is as follows:

The catheter balloon of the present invention comprising:
a cylindrical balloon part that is made non-expansive or low-expansive; and
a band part that is wound around the balloon part and is made of an elastic material having a shorter diameter than a spread diameter of the balloon part.

According to the present invention, the balloon part is made non-expansive or low-expansive; thus, the balloon can be prevented from continuing to be inflated to be excessively largely increased in diameter as seen in an expansive balloon. Consequently, the balloon can be prevented from being excessively spread to damage tissues therearound. When the band part is wound at the center, both sides of the band part (hereinafter referred to as "shoulder parts") form spread regions wherein the band part is not wound. When an appropriate size for valve cusp and LVOT is selected, only the shoulder parts at the both sides are spread and the band part is not spread, by spreading the balloon at the pressure or less at which the band part expands. The shoulder parts are made to form waist parts whose radii are smaller than the shoulder parts. As a result, the catheter balloon can be naturally arranged in a manner that annulus is sandwiched between the shoulder parts.

In the catheter balloon according to the present invention, the balloon part may be made of non-expansive material or low-expansive material. By adopting such a configuration, a non-expansive or low-expansive balloon can be easily manufactured.

In the catheter balloon according to the present invention, the expansion percentage of the outer diameter may be 10% or less, when the balloon part is inflated at the pressure of 0.2 atm. By adopting such a configuration, it is surely prevented from breaking the surrounding tissue due to the over-expansion of the balloon part.

In the catheter balloon according to the present invention, the balloon part may be inflated to form a substantial gourd shape with which the band part is not provided at less than a predetermined pressure, and the band part may also be inflated at the predetermined pressure or more. By adopting such a configuration, the inflated band part can dilate valve cusp to break its calcified part.

In the catheter balloon according to the present invention, the balloon part may be inflated to from a substantial gourd shape with which the band part is not provided at less than a predetermined pressure, and the band part may also be inflated to form a substantial cylindrical shape at the predetermined pressure or more. By adopting such a configuration, the band part can be spread to the same diameter as the shoulder parts to break the calcified part of valve cusp.

In the catheter balloon according to the present invention, the balloon part may be inflated to form a substantial gourd shape with which the band part is not provided at less than a predetermined pressure, and may be a gourd shape even if a pressure equal to or more than the predetermined pressure is applied. By adopting such a configuration, the possibility of the balloon part being freely shifted can be prevented at the time of breaking the calcified part of valve cusp.

In the catheter balloon according to the present invention, the material forming the inner surface of the band part and the material forming the outer surface of the balloon part may be compatible. By adopting such a configuration, the balloon part and the band part can be welded together to be fixed.

In the catheter according to the present invention, an outer tube and the catheter balloon communicated with the lumen for injecting the spreading fluid of the outer tube are comprised. With these, the catheter which has the above-mentioned effect can be provided.

The method of manufacturing the catheter balloon according to the present invention is comprising:
(1) a process of arranging a tubular band part around a tubular member made of non-expansive material or low-expansive material; and
(2) a process of heating these parts to extend the tubular member by heat and inflate it to a cylindrical shape.

According to such a manufacturing method, the catheter balloon can be manufactured in a single process. And the whole surfaces of the band part and the balloon part can be thermally welded.

The method of manufacturing the catheter balloon according to the present invention may comprise:
(1) a process of heating the tubular member made of non-expansive material or low-expansive material to extend it by heat and inflate it to a cylindrical shape.
(2) a process of attaching a expansive band to the processed cylindrical material.

In this case, the expansive band may be attached to the processed cylindrical material by adhesion bonding, thermal welding, and etc.

### Advantageous Effects of Invention

According to the catheter balloon, medical apparatus and the method of manufacturing the catheter balloon according to the present invention, the spreadable outer diameter of the balloon, which is expandable, can easily be controlled and further the balloon can be effectively prevented from being freely shifted in treatments for calcific, rheumatic and congenital aortic stenosis, and others. Moreover, the present invention can provide a catheter balloon, a catheter and a method of manufacturing the catheter balloon, this balloon and a balloon of this catheter being capable of being easily fixed at a predetermined position.

### Brief Description of Drawings

[Fig. 1] Fig. 1A shows a schematic view of a catheter balloon 10 according to an embodiment that shoulder parts thereof are spread; Fig. 1B shows a schematic view illustrating a state according to the embodiment that a balloon part and a band part are disassembled.
[Fig. 2] Fig. 2 shows variations of the catheter balloon 10 according to the embodiment that the band part 30 thereof is expanded.
[Fig. 3] Fig. 3 shows views illustrating a process of the first manufacturing method of the catheter balloon 10 according to the embodiment.
[Fig. 4] Fig. 4 shows an enlarged sectional view along the line B-B in Fig. 3.
[Fig. 5] Fig. 5 shows views illustrating a process of the second manufacturing method of the catheter balloon 10 according to the embodiment.
[Fig. 6] Fig. 6 shows views illustrating a process of a method using the catheter balloon 10 according to the embodiment.
[Fig. 7] Fig. 7 shows a graph indicating a change in the internal pressure of the balloon, and a change in the outer diameter of the catheter balloon.

### Embodiment Carrying Out the Invention

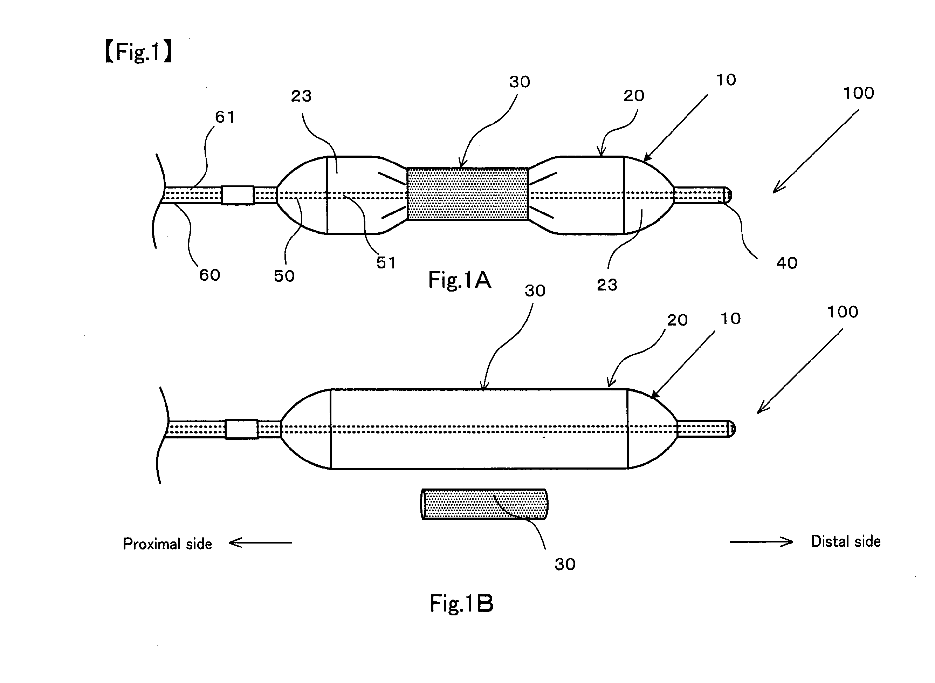

Hereinafter a detailed description will be made along with the drawings about a catheter balloon 10, a catheter 100, and a method of manufacturing the catheter balloon 10 according to an embodiment. Fig. 1 shows a schematic view of the catheter balloon that shoulder parts are spread according to the present invention. Fig. 1B shows a schematic view illustrating a state that a balloon part and a band part are disassembled. In the present specification, the word "spread" means that a balloon is developed merely by an increase in the internal pressure thereof but does not mean that the balloon itself is substantially expanded to be inflated. The word "expand" means that a band part is expanded to be made longer in diameter.

As illustrated in Fig. 1, the catheter balloon 10 according to the present embodiment comprises the balloon part 20 in a cylindrical shape made of non-expansive material or low-expansive material, and the band part 30 manufactured of stretching elastic body. The band part 30 has a shorter diameter than the spread diameter of the balloon part 20 in a state that a stretching force is not applied, and is wound around a middle position of the balloon part 20. Thus, the wound part by the band part 30 of a cylindrical part 21 of the balloon part 20 is not easily spread and is intentionally thinned down, and the band part 30 and a surrounding part thereof including the shoulder parts 23 are easily spread.

The balloon part 20 is formed in a hollow bag shape, whose middle part is formed in a cylindrical shape, and whose both ends are tapered and formed in a conical shape. The balloon itself is formed non-expansive or low-expansive, and formed not to be inflated more than a certain level even if the inside is pressurized. Various kinds of well-known materials may be used for the balloon part 20; for example, nylon, polyvinyl chloride, polyethylene, polyurethane, polyether block amide copolymer, polyethylene telephthalate, olefinic polymer such as polypropylene, or copolymer consisting of a combination thereof, and others. Preferably, non-expansive material or low-expansive material is recommended for the material forming the balloon part 20 itself. The balloon part 20 is not necessarily made of a single material, and may be a double layer consisting of different materials between an inner layer and an outer layer. The double layer balloon can be manufactured by shaping a tube manufactured by a double-layer extrusion molding. If a material compatible with a band part 30 that will be described later is used for the outer layer, for instance, it can be welded with the band part 30 so that they are easily fixed each other. By adopting the above-mentioned expansive percentage, over-spreading can be prevented. The balloon part 20 is formed in length from 30 mm to 80 mm, preferably from 50 to 70 mm, and the maximum spreading diameter is formed, preferably, from 10 mm to 25 mm. Depending on the size of the maximum spreading diameter, it can be applied to the openings of a body and other hollow organs in addition to artery and vein.

The band part 30 is an expansive ring member, which is wound around the balloon part 20 to keep a part of the balloon part 20 less than the maximum spreading diameter at a certain pressure or less, and to be expanded at the certain pressure or more. Silicone, polyurethane, other elastomer-based polymer, and others, are used accordingly for materials, however, are not limited for the materials. The thickness of the band part 30 is manufactured from 20 µm to 100 µm, preferably from 50 to 70µm, the width is manufactured from 3 to 30 mm, preferably 5 to 15 mm, and the diameter is manufactured from 3 mm to 20 mm. The diameter must be smaller than the maximum spreading diameter of the balloon part 20.

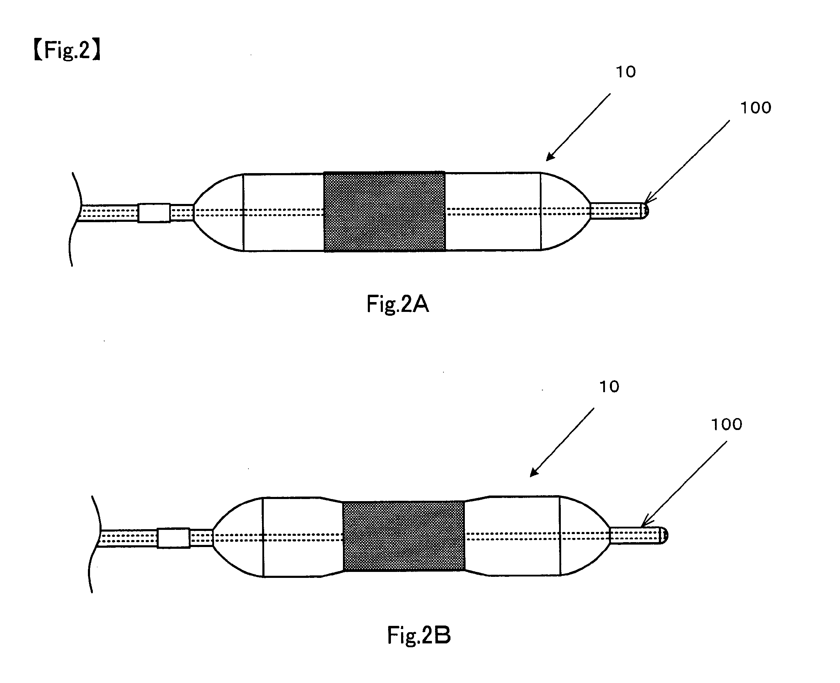

About the catheter balloon 10 formed in the above-mentioned manner, the band part 30 keeps the diameter of a part of the balloon part 20 small. Thus, when the spreading fluid is inserted into the balloon 20 at a predetermined pressure or less, specifically at a pressure permitting a waist part 30 to be spread or inflated, or lower, only the shoulder parts 23 are being spread while the waist part keeps the initial diameter thereof. Subsequently, the spread of the shoulder parts 23 and the other reaches into an equilibrium. The spread of the shoulder parts 23 causes the balloon 20, which has been folded up, to merely open, and thus the raw material itself of the balloon 20 is not expanded. Accordingly, the shoulder parts 23 are spread even at a relatively low pressure, for example, 0.05 atm or less. However, about the band part 30, the raw material itself thereof needs to be expanded. Thus, at a low pressure, the band part 30 is hardly expanded so that the band part 30 is kept to have the pre-expansion diameter. Consequently, as illustrated in Fig. 1A, at the fluid pressure or less which the band part 30 is expanded, it is formed in a so-called gourd shape as a whole, which is such a shape that the waist part 22 of the center of the balloon part 20 is narrow and the shoulder parts 23 are thick. When a valve cusp or annulus is fitted onto this waist part, the balloon part is easily arranged at a predetermined position so that the balloon part can be prevented from being freely shifted. In this state, the band part 30 arranged at the waist part 22 is expanded and increased in diameter, by means of injecting the spreading fluid to increase the internal pressure of the balloon part 20. At this time, the shoulder parts 23 of the balloon part 20 are non-expansive or low-expansive, and thus after the equilibrium state, the shoulder parts 23 are not spread or are slightly spread. The spreading fluid is further injected to increase the inner pressure of the balloon part 20 and expand the band part 30, and then the diameter of the balloon part 20 of the waist part 22 is being expanded. As a result, the waist part 22 of the balloon part 20 is spread to the same degree as the diameter of the shoulder parts 23. Because the balloon part 20 is non-expansive or low-expansive, the balloon part 20 is merely spread slightly more largely. It is therefore possible to prevent the diameter from continuing to be inflated and being exploded easily such as an expansive balloon. At this time, the spreading pressure varies depending on the width, thickness, circumferential length or materials of the band part 30. By adjusting these factors, as illustrated in Fig. 2B, the waist part 22 of the balloon part 20 may be adjusted to be slightly smaller in diameter than the shoulder parts 23. By adopting such an adjustment, it is possible to prevent the catheter balloon 10 from being freely shifted.

Next, a method of manufacturing the above-mentioned catheter balloon 10 is described.

### (The first manufacturing method)

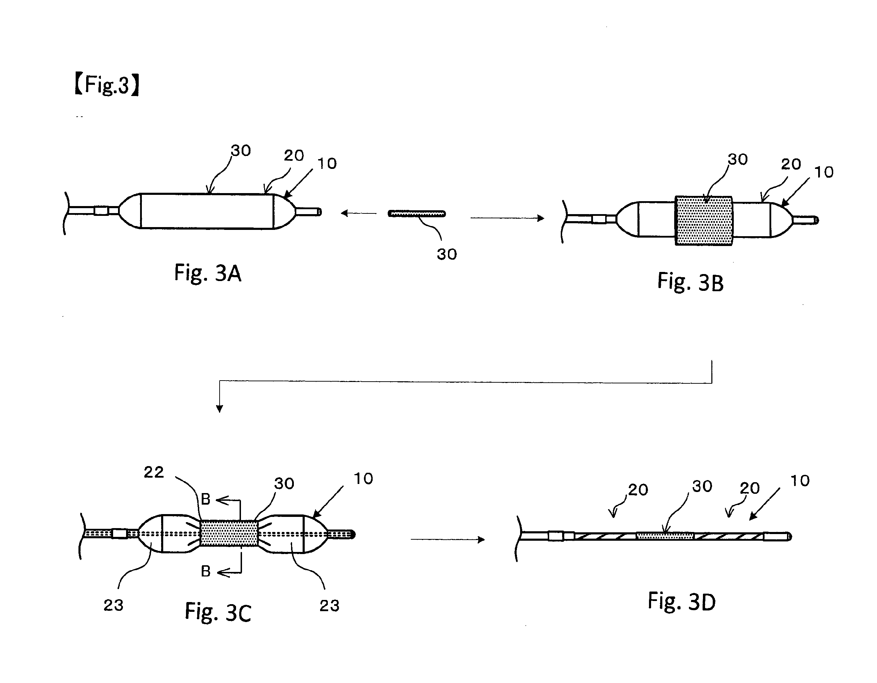

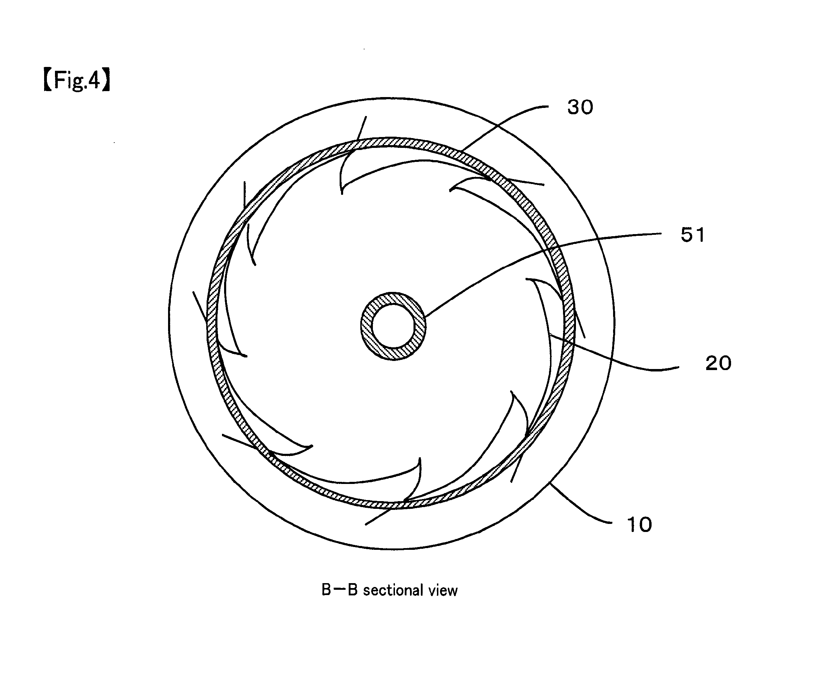

The first manufacturing method is a method of manufacturing the balloon part 20 first, and then the band part 30 is attached with the balloon part 20. As a method of manufacturing the balloon part 20, blow-forming, or stretching a tube manufactured by extrusion-forming by spreading it after heating the tube, may be used. On the other hand, a method of manufacturing the band part 30 is not particularly limited, as long as a strip-like expansive sheet is prepared. For example, stretching a tube prepared by extrusion-forming or by dipping may be adopted. When the band part 30 is fixed onto the manufactured balloon part 20, the sheet-like band part 30 may be wrapped around the balloon part 20 and fixed, or the band part 30 preformed in a ring shape, as illustrated in Fig. 3A, may be inserted into the balloon part 20 and fixed as illustrated in Fig. 3B. Adhesion or welding can be selected as a fixing method of the band part 30 to the balloon part 20, however, not limited thereto. When welding by heat is selected, it is preferable to use a compatible material each for the balloon part 20 and the band part 30. At this time, as mentioned above, a compatible material may be used only for the outer layer of the balloon part 20. By adopting such a double layered structure, expansive performance and welding performance can be allocated to each layer so that each physical property is easily satisfied. The part of which the band part 30 is fixed to the balloon part 20 may be fixed to the front face of the balloon part 20, or as illustrated in Fig. 4, when the balloon part 20 is folded in a wing-like fashion (folded in a so-called umbrella shape), the part may be evenly-fixed to the front end or middle part of each wing. With this evenly-fixing method, the waist part 22 can be prevented from being unevenly spread as much as possible. As illustrated in Fig. 3C, the balloon part 20 fixed with the band part 30 is formed in a shape of which the waist part is thin and the shoulder parts 23 are thick like a gourd. Then it is completed, as illustrated in Fig. 3D, by folding in a winding manner the shoulder parts 23.

### (The second manufacturing method)

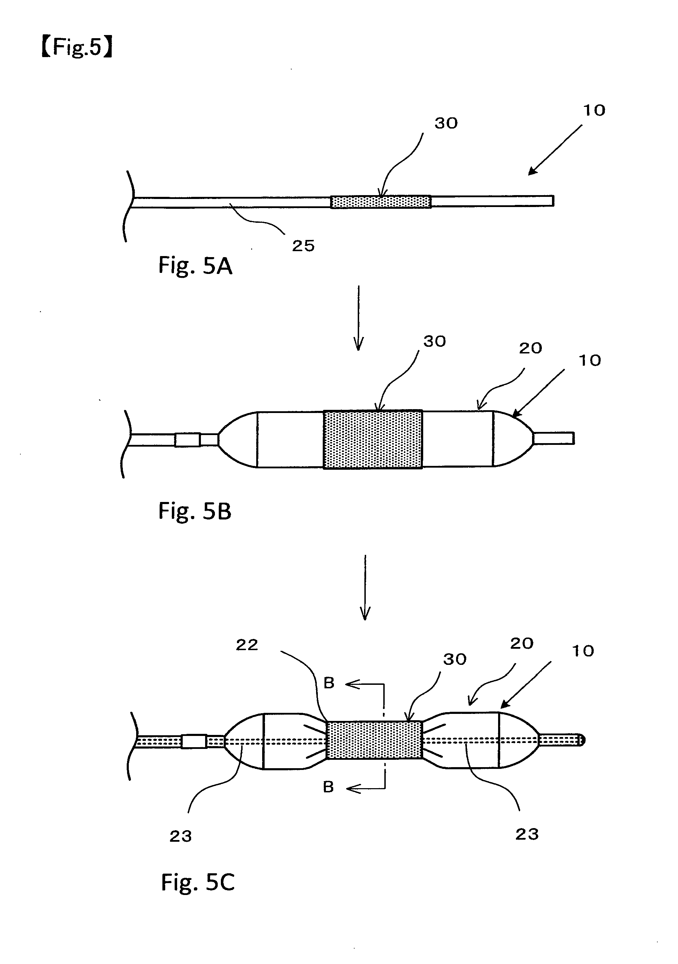

The second manufacturing method is a method of forming both the balloon part 20 and band part 30 at the same time by blow-forming. First, as illustrated in Fig 5A, a tube that is the material of the band part 30 is fixed around a parison 25 that is a tubular member before blow-forming. In this state, as illustrated in Fig. 5B, the band part 30 with the balloon part 20 is spread and extended by blow-forming, and as illustrated in Fig. 5C, is adhered together by heat at the time of the forming. By adopting such a method, the catheter balloon 10 can be manufactured only by one process. And it becomes easy for the band part 30 and the balloon part 20 to adhere together on the entire surfaces.

The balloon part 20 manufactured in the above-mentioned manner is arranged at the distal end of a catheter main body to be a catheter. Specifically, as illustrated in Fig. 1A, the catheter comprises a tip part 40 installed at the distal end of the catheter main body, an inner tube 50 having a guide wire lumen 51 connected with the tip part 40, the balloon part 20 arranged around the guide wire lumen 51, and an outer tube 60 having a spreading-fluid-injecting lumen 61 communicated with a balloon lumen 52 of the balloon part 20. The balloon part 20 is inflated by the spreading fluid through the spreading-fluid-injecting lumen 61.

The following will describe how to use the above-mentioned catheter for valvuloplasty. According to the Seldinger technique, the catheter 100 is introduced through a thigh, upper arm artery or left subclavian artery. Alternatively, after a surgical incision, the catheter 100 is directly inserted into an aortic valve, so that the catheter 100 can be anteogradely inserted. Hereinafter, as an example, a case will be described where at the time of a detention of the catheter balloon 10 in a transcatheter aortic valve replacement, the catheter 10 is detained in a valve cusp by an approach through a thigh. It is however needless to say that various detaining techniques are usable in accordance with the present invention.

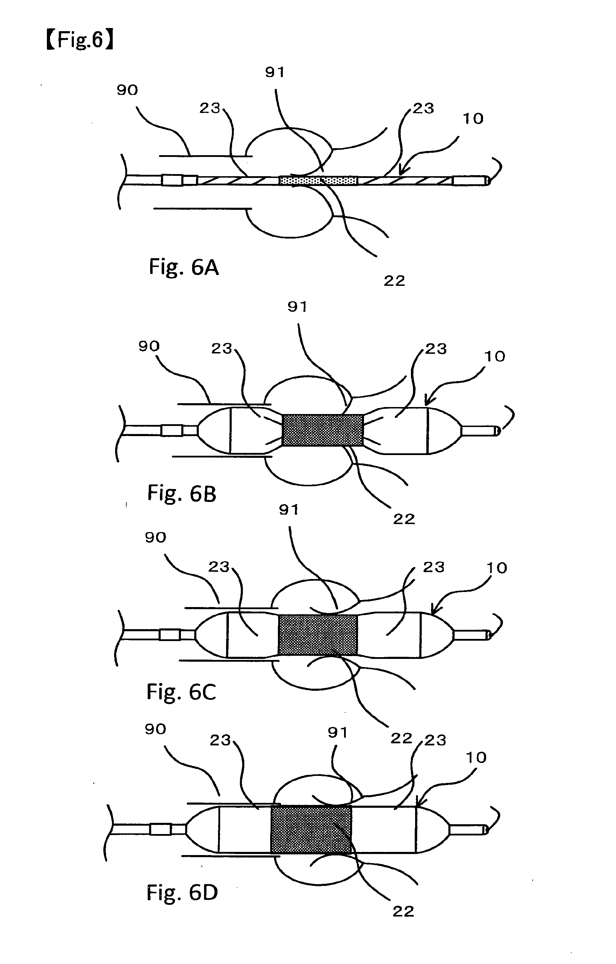

A puncture needle is stuck into a femoral artery (or an iliac artery inside the pelvis), which is near person's groin. Thereafter, a guide wire is inserted into the blood vessel. While the guide wire is being shifted along the blood vessel, the guide wire is slipped to be inserted into the blood vessel. The guide wire is passed through the aortic valve, and then indwelled. Thereafter, while the position of the guide wire is being monitored on a roentgenoscopic screen, the balloon 10 for catheter is retrogradely advanced to a target position. Next, as illustrated in Fig. 6A, the catheter balloon 10 is detained to position the shoulder part 23 at the distal end side of the balloon 10 inside the left ventricule outflow tract, and position the waist part 22 at a valve cusp 91 of an aorta 90.

As illustrated in the drawing 6B, a spreading fluid is introduced into the catheter balloon 10 to increase the pressure therein up to about 0.1 atm to 0.5 atm (the pressure in the balloon part 20 is made slightly higher than that outside the balloon). In this way, the balloon 20 is spread. According to this pressure, the shoulder part 23 at the proximal side of the catheter and the shoulder part 23 at the distal side are spread while the waist part 22 is not spread to keep the diameter thereof small. In this state, about the catheter balloon 10, the spreadable diameter of the shoulder parts 23 is selected into a size suitable for the valve cusp and the LVOT. For this reason, the annulus is arranged to be sandwiched between the shoulder parts 23 at both the sides, so that the waist part 22 is arranged naturally at the valve cusp. Consequently, the balloon catheter 10 can be arranged at a desired position to be prevented from being freely shifted.

Next, the pressure inside the balloon part 20 is further increased, and the spreading fluid is introduced thereinto to give a pressure in the range of 0.5 atm to 5.0 atm, preferably 1.0 atm to 3.0 atm. As a result, the waist part 22 is spread to start pushing and moving the valve cusp outward. The spread of the waist part 22 pushes and moves the valve cusp to be opened, so that its calcified part is pulverized.

As the pressure inside the balloon part 20 is further increased, the diameter of the waist part 22 is gradually increased. Thus, as illustrated in Fig. 6D, the waist part 22 is spread into an outer diameter equivalent to that of the shoulder parts 23 which the band part 30 is not installed. Since the outer diameter of the waist part 22 is manufactured in the same size as that of the shoulder parts 23, it is not further spread and can be prevented completely from being excessively spread and damaging tissues around the balloon. Since the blockage of the blood flow is restricted for a short period of time, the balloon part 20 is deflated in a short period. When the balloon is spread, a contrast medium may be injected into the balloon part 20. In this way, it is possible to roentgenize the valve cusp in the state that the valve cusp is spread. Thus, the diameter of the spread valve cusp can be estimated.

### (Example)

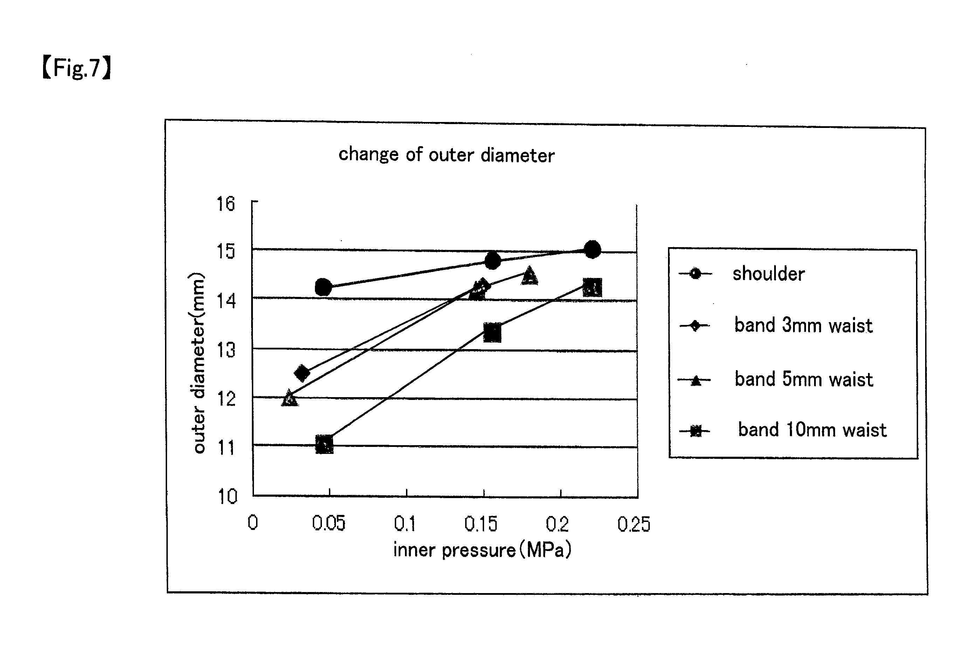

Hereinafter, experimental results are shown about the relationship between the pressure and the outer diameter in a working example. The present example merely shows one aspect of the invention. Thus, raw material and forms usable in the present invention are not limited to the present raw materials and form. The band parts 30 which are manufactured of polyurethane as an expansive material, 9 mm in the outer diameter, and 3mm, 5 mm, and 10 mm each in width is prepared and attached with the balloon parts 20 which are manufactured of low-expansive nylon and 15 mm in the outer diameter. By injecting a spreading fluid into the balloon part 20, a change is measured in the inner pressure of the balloon part 20 and the outer diameter of the waist part 22 and shoulder parts 23 attached with the band part 30. The measurement of the outer diameter of each of the parts is started when the pressure begins rising inside the balloon part 20 after a spreading fluid is injected. Thereafter, the pressure and the outer diameter are measured each time a predetermined amount of the fluid is injected. Because the pressure is decreasing over time due to the creep phenomenon of the expansive material of the band part 30, the pressure in stable condition is recorded after a certain amount of time has been passed. The measurement result is as follows; with the 3 mm band width, 12.6 mm at the pressure of 0.032 MPa, and 14.3 mm at the pressure of 0.150MPa. With the 5 mm band width, 12.0 mm at the pressure of 0.024 MPa, 14.2 mm at the pressure of 0.1450 MPa, and 14.5 mm at the pressure of 0.180 MPa. With the 10 mm band width, 11.1 mm at the pressure of 0.046 MPa, 13.4 mm at the pressure of 0.156 MPa, and 14.3 mm at the pressure of 0.221 MPa. With the shoulder parts, on the other hand, 14.3 mm at the pressure of 0.046 MPa, 14.8 mm at the pressure of 0.156 MPa, and 15.1 mm at the pressure of 0.221 MPa. The graph of the results is shown in the Fig. 7.

According to the measurement results, a higher pressure than the expansivee strength of the band part 30 is necessary to expand the band part 30. However, this pressure can be changed depending on the material and size, etc. of the band part 30. The diameter of the shoulder parts 23 inflates slightly along with the pressure increase, however, non-expansive material is used and 10% or less of the inflation rate is achieved so that no big difference can be seen. While the pressure is increasing to expand the band part 30, and the shoulder parts 23 are getting close to the outer diameter, however, depending on the material or the size of the band part 30, the band part 30 cannot be the same as the outer diameter of the shoulder parts 23. It shows the slightly smaller diameter than that of the shoulder parts 23. As a result, an uneven shape of the balloon part 20 can prevent the balloon part 20 from slipping out of the valve and shifting freely.

The present invention is not limited to the above-mentioned embodiment, and a variety of embodiments can be practiced within the technical scope of the present invention.

First, the catheter balloon 10 and the catheter 100 according to the present embodiment were described through an example of a transcatheter aortic valve replacement; however, they can be applied to the valvuloplasty of a stenotic aortic valve or pulmonary valve, or for dilating all stenotic constrictions in blood vessels. They also can be applied to the pre-spread of aortic valve cusp before placing a percutaneous aortic valve or any other artificial device used for aortic valve repair, replacement or transplantation. The applicable blood vessels include all blood vessels of the body, such as coronary arteries, peripheral arteries, veins, the gullet, the trachea, enteric blood vessels, the bile duct and the uriter, etc. of the body.

### Industrial Applicability

As has been demonstrated in the above-mentioned embodiment, the present invention is usable for intervention operations, especially for preventing a blood clot or debris from being scattered.

### Description of Reference Signs

- 10:: catheter balloon
- 20:: balloon part
- 21:: cylindrical part
- 22:: waist part
- 23:: shoulder part
- 25:: parison
- 30: band part
- 40:: tip part
- 50:: inner tube
- 51:: guide wire lumen
- 52:: balloon lumen
- 60:: outer tube
- 61:: spreading-fluid-injecting lumen
- 90:: aorta
- 91:: valve cusp
- 100:: catheter

## Claims

1. A catheter balloon, comprising:
a cylindrical balloon part that is made non-expansive or low-expansive, and
a band part that is wound around the balloon part and is made of an elastic body having a shorter diameter than a spread diameter of the balloon part.

2. The balloon according to claim 1, wherein only the balloon part is inflated to form a substantial gourd shape with which the band part is not provided at less than a predetermined pressure, and
only the band part is inflated to increase its diameter and form a substantial cylindrical shape at the predetermined pressure or more.

3. The catheter balloon according to claim 1 or 2, wherein only the balloon part is inflated to form a substantial gourd shape with which the band part is not provided at less than a predetermined pressure, and
the balloon part is a gourd shape even if a pressure equal to or more than the predetermined pressure is applied.

4. The catheter balloon according to any one of claims 1 to 3, wherein the material forming the inner surface of the band part and the material forming the outer surface of the balloon are compatible.

5. A catheter, comprising:
an outer tube, and
the catheter balloon according to any one of claims 1 to 4, which is communicated with the lumen for injecting the spreading fluid of the outer tube.

6. A method of manufacturing the catheter balloon according to claim 1 to 4, comprising the steps of:
(1) arranging a tubular band part around a tubular member made of non-expansive or low-expansive material, and
(2) heating these to extend the tubular member by heat and inflate it to a cylindrical shape.
